# EUROPÄISCHE PATENTSCHRIFT

(11) **EP 1 223 172 B1**
(45) Veröffentlichungstag und Bekanntmachung des Hinweises auf die Patenterteilung: **13.10.2004**
(21) Anmeldenummer: 01129790.0
(22) Anmeldetag: 14.12.2001
(51) Int. Cl.: C07D 487/08

(54) **Verfahren zur Herstellung von reinem Triethylendiamin (TEDA)**
Process for the production of pure triethylene diamine (TEDA)
Procédé de préparation de triéthylènediamine (TEDA) pure

(30) Priorität: 10.01.2001 DE 10100943
(43) Veröffentlichungstag der Anmeldung: 17.07.2002
(73) Patentinhaber: BASF AKTIENGESELLSCHAFT, 67056 Ludwigshafen (DE)
(72) Erfinder: Lang, Ortmund, 66909 Quirnbach (DE); Rumpf, Bernd, Dr., 68766 Hockenheim (DE); Frauenkron, Matthias, Dr., 67061 Ludwigshafen (DE); Funhoff, Dirk, Dr., 68165 Mannheim (DE); Manderbach, Thomas, Dr., 67063 Ludwigshafen (DE); Stein, Bernd, Dr., 64342 Seeheim-Jugenheim (DE)

(56) Entgegenhaltungen:
- EP-A- 1 070 717
- DE-A- 19 933 850
- DE-A- 19 962 455
- GB-A- 2 080 283
- US-A- 902 073
- US-A- 3 993 651
- US-A- 4 182 864
- US-A- 4 216 323
- CHEMICAL ABSTRACTS, vol. 81, no. 19, 1974 Columbus, Ohio, US; abstract no. 120698, MURAKAMI: "distillation of piperazine and triethylenediamine" XP002199302 & JP 49 048609 A (SHUNAN PETROCHEMICAL CO.) 11. Mai 1974 (1974-05-11)

## Beschreibung

Die vorliegende Erfindung betrifft ein Verfahren zur Herstellung von reinem Triethylendiamin (= TEDA = DABCO®= 1,4-Diazabicyclo-[2,2,2]-octan) und Lösungen hiervon.

Triethylendiamin (TEDA), das unter Normalbedingungen ein Feststoff ist, ist ein bedeutender Katalysator für die Herstellung von Polyurethanschäumen.

Für diese und andere Einsatzgebiete ist ein reines, möglichst geruchloses und reinweißes TEDA mit einer möglichst geringen Verfärbung, z. B. mit einer möglichst kleinen APHA-Farbzahl (DIN-ISO 6271), das diese Eigenschaften auch über längere Lagerzeiten (von z. B. 6, 12 oder mehr Monaten) beibehält, erwünscht.

Zur Herstellung und Reinigung von TEDA sind verschiedene Verfahren bekannt:

DT-A-24 42 929 betrifft ein Verfahren zur Herstellung von TEDA durch Abspaltung von Glykol aus N,N'-Di(hydroxyethyl)piperazin in Gegenwart von Al₂O₃ als Katalysator.

US-A-3,297,701 offenbart ein Verfahren zur Herstellung von Diazabicyclo-[2,2,2]-octanen durch Umsetzung entsprechender Hydroxyethyl- oder Aminoethyl-piperazine bei erhöhter Temperatur in Gegenwart von Metallphosphaten, wie z. B. Calciumphosphat.

DE-A-36 34 258 beschreibt ein Verfahren zur Herstellung von Diazabicyclo-[2,2,2]-octanen durch Umsetzung entsprechender Hydroxyethyl- oder Aminoethyl-piperazine in Gegenwart von Zirkoniumphosphaten.

DE-A-1 745 627 betrifft ein Verfahren zur Herstellung von TEDA und Piperazin durch Umsetzung eines Ethylendiamins an einem sauren Kieselsäure-Tonerde-Katalysator bei erhöhter Temperatur und Gewinnung des TEDAs durch Destillation und/oder Kristallisation.

DE-A-37 18 395 beschreibt die Herstellung von TEDA durch Umsetzung eines acyclischen Hydroxyethyl-ethylenpolyamins und/oder cyclischen Hydroxyethyl-ethylenpolyamins in Gegenwart eines phosphorhaltigen Titandioxid- oder Zirkoniumdioxid-Katalysators.

EP-A-111 928 beschreibt die Verwendung von bestimmten Phosphatkatalysatoren, wie z. B. Mono- oder Pyrophosphate des Magnesiums, Calciums, Bariums oder Aluminiums, bei organischen Kondensationsreaktionen, wie z. B. der Umsetzung von N-(2-Hydroxyethyl)-piperazin zu TEDA.

EP-A-382 055 offenbart ein Verfahren zur Herstellung von TEDA, wobei man 1,2-Diaminoethan und 0 bis 200 Mol-% Piperazin an Al-, B-, Ga- und/oder Fe-silikatzeolithen bei erhöhter Temperatur umsetzt.

EP-A-842 935 beschreibt ein Verfahren zur Herstellung von TEDA durch Umsetzung einer Aminverbindung, wie Monoethanolamin, an einem Katalysator zu einem Produkt enthaltend TEDA und Piperazin und anschließende Umsetzung dieses Produkts mit einer ethylierenden Verbindung, die zumindest ein N und/oder O-Atom enthält, in Gegenwart eines formselektiven Zeolithkatalysators.

US-A-5,741,906 betrifft die Herstellung von TEDA durch Umsetzung einer Aminverbindung, wie Monoethanolamin, an einem Zeolithkatalysator vom Pentasil-Typ.

Die bekannten Verfahren zur TEDA-Herstellung führen zur Bildung roher Umsetzungsprodukte, die neben TEDA noch Wasser, Nebenprodukte, wie z. B. Piperazin und hochmolekulare Polymerisate, sowie ein gegebenenfalls bei der Umsetzung eingesetztes Lösungsmittel enthalten. TEDA wird aus diesen Gemischen gewöhnlich durch diskontinuierliche oder kontinuierliche Destillation oder Rektifikation abgetrennt und meist in einem anschließenden Schritt durch Kristallisation oder Umkristallisation gereinigt.

TEDA ist aufgrund seiner Eigenschaften [hygroskopisch, temperaturempfindlich, Siedepunkt (174 °C bei Normaldruck) und Schmelzpunkt (158-160 °C) liegen dicht beieinander] schwierig und nur unter entsprechendem technischem Aufwand zu handhaben, ohne dass eine Verschlechterung der Qualität des TEDAs bezüglich Farbe, Farbstabilität (unerwünschte Zunahme der Farbzahl, z. B. gemessen als APHA-Farbzahl, über die Lagerzeit), Geruch (unerwünschter Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen) und Reinheit auftritt.

Das nach den bekannten Verfahren nach einer Destillation oder Rektifikation erhaltene TEDA und hieraus hergestellte Lösungen ist/sind aufgrund der Farbe (z. B. gemessen als APHA-Farbzahl), Farbstabilität und/oder des Geruches meist nicht marktfähig und nur eine oder mehrere weitere Reinigungsstufe(n), wie z. B. eine technisch aufwendige, zum Teil mehrstufige, Kristallisation oder Umkristallisation, kann die TEDA-Qualität verbessern.

Es hat daher nicht an Versuchen gefehlt, alternative Verfahren aufzufinden, die TEDA in verbesserter Qualität bereitstellen.

DT-A-26 11 069 betrifft die Gewinnung von TEDA, wobei man dem rohen TEDA Propylenglykol zusetzt und anschließend fraktionierend destilliert.

DE-A-28 49 993 offenbart ein Verfahren zur Abtrennung und Gewinnung von TEDA, wobei man dem rohen TEDA Wasser zusetzt und anschließend destilliert.

JP-A-49 048 609 beansprucht ein Verfahren zur Reinigung von Piperazin und/oder TEDA durch fraktionierte Destillation einer Mischung enthaltend Piperazin und/oder TEDA, umfassend die Schritte Auflösen der Piperazin- und/oder TEDA-Destillate in Wasser oder einem organischen Lösungsmittel, wobei das Lösungsmittel flüssig oder gasförmig vorliegen kann, und Sammeln der Lösungen der Destillate. Mit diesem Verfahren soll anmeldungsgemäß die Aufgabe gelöst werden, Verstopfungen durch Feststoffe in der Destillationsapparatur zu verhindern. Die Beschreibung, die schematischen Darstellung der Destillationsapparatur und die Beispiele in dieser Patentanmeldung lehren, dass hierzu das Piperazin oder das TEDA am Kopf der Destillationskolonne in einem Kondensator zunächst verflüssigt und erst danach in dem Lösungsmittel aufgelöst wird.

Nachteilig an diesen Verfahren ist, dass sie das TEDA nicht in der erwünschten Qualität liefern.

Die älteren deutschen Patentanmeldungen Nr. 19933850.7 vom 23.07.99 und Nr. 19962455.0 vom 22.12.99 betreffen ein Verfahren zur Herstellung einer Lösung von reinem TEDA, wobei man TEDA verdampft und das dampfförmige TEDA in ein flüssiges Lösungsmittel einleitet, sowie ein Verfahren zur Herstellung von reinem TEDA, indem man das TEDA aus dieser Lösung auskristallisiert.

Der vorliegenden Erfindung lag die Aufgabe zugrunde, ein verbessertes, effizientes und wirtschaftliches Verfahren zur Herstellung von reinem Triethylendiamin (TEDA) und Lösungen hiervon aufzufinden, das TEDA und TEDA-Lösungen mit verbesserter Qualität bezüglich Farbe, Farbstabilität, Geruch und Reinheit liefert.

Demgemäß wurde ein Verfahren zur Herstellung einer Lösung von reinem Triethylendiamin (TEDA) gefunden, welches dadurch gekennzeichnet ist, dass man TEDA aus einer Mischung enthaltend ein Lösungsmittel oder ein Verdünnungsmittel, wobei das Lösungs- oder Verdünnungsmittel bei Normaldruck (= 1,01325 bar) einen Siedepunkt im Bereich von 175 bis 250 °C aufweist, verdampft und das dampfförmige TEDA in ein flüssiges Lösungsmittel einleitet.

Durch anschließende Auskristallisation des TEDAs aus der so erhaltenen Lösung wird reines TEDA mit der aufgabengemäß verbesserten Qualität erhalten.

Bei dem Lösungsmittel, in dessen Gegenwart das TEDA verdampft wird und dem Lösungsmittel, in das das dampfförmige TEDA eingeleitet wird, kann es sich um das gleiche oder auch um verschiedene Lösungsmittel handeln.

Durch das erfindungsgemäße Verfahren, wobei die Einleitung des dampfförmigen TEDAs in ein flüssiges Lösungsmittel im Folgenden auch TEDA-Quench genannt wird, wird die Bildung von unerwünschten Neben- und Zersetzungsprodukten, die zur Qualitätsminderung des TEDAs führen, entscheidend verringert.

Der flüssige Aggregatzustand des TEDAs am Ausgang der Verdampfungsapparatur, z. B. Rektifikations- oder Destillationsapparatur, wird erfindungsgemäß vermieden, die bei Destillationen übliche Verflüssigung des Destillats findet nicht statt. Das dampfförmige TEDA wird stattdessen direkt in ein flüssiges Lösungsmittel eingeleitet.

Das Lösungs- oder Verdünnungsmittel, das die Mischung enthält, aus der das TEDA verdampft wird, weist bevorzugt bei Normaldruck einen Siedepunkt im Bereich von 180 bis 250 °C, besonders im Bereich von 180 bis 230 °C, insbesondere im Bereich von 190 bis 210 °C, auf.

Als Lösungs- oder Verdünnungsmittel, das die Mischung enthält, aus der das TEDA verdampft wird, eignen sich besonders inerte
- polare aprotische Lösungsmittel [z. B. N-Alkyl-2-pyrrolidone (wie N-Methyl-2-pyrrolidon (NMP), 1-Ethyl-2-pyrrolidon, 1,5-Dimethyl-2-pyrrolidon, 1-Isopropyl-2-pyrrolidon), Ether (wie Diethylenglykoldiethylether, Triethylenglykoldimethylether, Triethylenglykoldiethylether), Ketone (wie Acetophenon, Propiophenon), Lactone (wie γ-Butyrolacton), Sulfoxide (wie Dimethylsulfoxid), Carbonsäureester (wie Fumarsäuredimethylester), Nitrile (wie Benzonitril) und Harnstoffe (wie 1,3-Dimethyl-imidazolidin-2-on (DMEU), Tetramethylharnstoff)],
- cyclische oder acyclische Kohlenwasserstoffe, insbesondere gesättigte cyclische oder acyclische Kohlenwasserstoffe (z. B. Undecan, Dodecan, cis-Dekalin, trans-Decalin),
- chlorierte aliphatische Kohlenwasserstoffe (z. B. 1-Chloroctan, 1,1-Dichloroctan),
- aromatische Kohlenwasserstoffe, Nitroaromaten und Phenole (z.B. Naphthalin, n-Butylbenzol, Phenol, Kresol, Nitrobenzol, Nitrophenol),
- chlorierte aromatische Kohlenwasserstoffe (z. B. 1,2-Dichlorbenzol, Benzylchlorid, 1,2,3,4-Tetramethylbenzol, 1,2,3,5-Tetramethylbenzol),
- Alkohole (z. B. Benzylalkohol, 2-Ethylhexanol, 1-Octanol, i-Decanol, 1,2-Propandiol, 1,3-Propandiol, Ethylenglykol, Diethylenglykol, 1,2-Propylenglykol, 1,3-Propylenglykol, 1,2-Butandiol, 1,3-Butandiol, 2,3-Butandiol, 1,4-Butandiol, Neopentylglykol, Diethylenglykolmonomethylether, Dipropylenglykol),
- primäre, sekundäre und tertiäre Amine (z. B. Tri-n-Butylamin, Benzylamin, Anilin, N-Ethylanilin, N,N-Dimethylanilin, N,N-Diethylanilin)
- N-Alkylamide (z. B. N-Methylformamid, N-Methylacetamid)
und deren Gemische.

Besonders bevorzugt sind polare aprotische Lösungs- oder Verdünnungsmittel mit einem E^{N}_{T}-Wert von 0,1 bis 0,6, besonders von 0,2 bis 0,5, insbesondere von 0,3 bis 0,45.

(Zur Definition des E^{N}_{T}-Wertes siehe Ch. Reichardt, Solvents and solvent effects in organic chemistry, 2. Auflage, VCH 1988).

Ganz besonders bevorzugte Lösungsmittel sind NMP und Ethylenglykol.

Das Lösungs- oder Verdünnungsmittel, das die Mischung enthält, aus der das TEDA verdampft wird, wird bevorzugt nach der Synthese des TEDAs dem rohen oder dem noch verunreinigten TEDA zugesetzt.

Vorteilhaft ist die Zugabe des Lösungsmittels in den Sumpf der Kolonne der TEDA-Destillation.

Das Lösungs- oder Verdünnungsmittel kann im einmaligen Durchlauf oder nach der Abtrennung der Schwersieder als Kreislauflösung eingesetzt werden.
Die Menge des verwendeten Lösungs- oder Verdünnungsmittel ist nicht erfindungswesentlich und wird nach Zweckmäßigkeitsgesichtspunkten ausgewählt. Im allgemeinen wird so verfahren, dass, je nach Art des Lösungs- oder Verdünnungsmittels, Lösungen oder Mischungen mit einem TEDA-Gehalt von ca. 1 bis 90 Gew.-%, bevorzugt 40 bis 70 Gew.-%, erhalten werden.

Die erfindungsgemäße Verdampfung des TEDAs aus einer Mischung enthaltend ein Lösungs- oder Verdünnungsmittel, kann nach den dem Fachmann geläufigen Verfahren und Bedingungen erfolgen, z. B. in einer Destillations- oder Rektifikationsapparatur, wobei das TEDA oder ein Gemisch enthaltend das TEDA (rohes TEDA) jeweils zusammen mit dem Lösungs- oder Verdünnungsmittel vorgelegt wird.

Bevorzugt wird das dampfförmige TEDA am Kopf oder in einem Seitenabzug einer Destillationskolonne erhalten. Das dampfförmige TEDA im erfindungsgemäßen Verfahren besitzt im allgemeinen eine Reinheit von größer 90 Gew.-%, bevorzugt größer 95 Gew.-%, insbesondere größer 97 Gew.-%.

Vorteilhaft wird bei der destillativen Aufarbeitung des TEDAs durch konstruktive Maßnahmen an Kolonnen und/oder Verdampfern (z. B.: Minimierung des Sumpfraumes) und/oder durch den Einsatz von thermisch schonenden Eindampfverfahren (z. B.: Fallfilmverdampfer, Dünnschichtverdampfer) die Verweilzeit und dadurch die thermische Belastung gering gehalten.

Im allgemeinen wird die Temperatur der Mischung, enthaltend TEDA und das Lösungs- oder Verdünnungsmittel, aus der das TEDA erfindungsgemäß verdampft wird (z. B. des Sumpfes der entsprechenden TEDA-Destillationskolonne), durch Wahl des einzusetzenden Lösungs- oder Verdünnungsmittels, des TEDA-Gehalts der Mischung und/oder des Drucks, auf ≤ 230 °C, bevorzugt 190 bis 210 °C eingestellt. Der Absolutdruck beträgt hierbei im allgemeinen 0,1 bis 5 bar, bevorzugt 0,5 bis 1,5 bar.

Die Zeitdauer zwischen Anfall des im erfindungsgemäßen Verfahren verwendeten dampfförmigen TEDAs und TEDA-Quench beträgt vorteilhafterweise ≤ 10 Sekunden.

Als Lösungsmittel für den TEDA-Quench eignen sich besonders cyclische oder acyclische (= aliphatische) Kohlenwasserstoffe (insbesondere verzweigte oder unverzweigte Alkane oder Alkangemische, wie z. B. n-Pentan, iso-Pentan, Cyclopentan, Hexan, Cyclohexan, Heptan, Octan, Petrolether), chlorierte aliphatische Kohlenwasserstoffe (insbesondere chlorierte Alkane, wie z. B. Dichlormethan, Trichlormethan, Dichlorethan, Trichlorethan), aromatische Kohlenwasserstoffe (wie z. B. Benzol, Toluol, Xylole), chlorierte aromatische Kohlenwasserstoffe (wie z. B. Chlorbenzol), Alkohole (wie z. B. Methanol, Ethanol, Ethylenglykol, 1,4-Butandiol, und Polyetheralkohole, insbesondere Polyalkylenglykole, wie Diethylenglykol, Dipropylenglykol), Ketone (insbesondere aliphatische Ketone, wie Aceton, Methylethylketon, Diethylketon), aliphatische Carbonsäureester (wie z. B. Essigsäuremethylester, Essigsäureethylester), aliphatische Nitrile (wie z. B. Acetonitril, Propionitril), Ether (wie z. B. Dioxan, THF, Diethylether, Ethylenglykoldimethylether) und deren Gemische.

Zur erfindungsgemäßen Herstellung einer Lösung von reinem TEDA, die z. B. als Katalysatorlösung bei der Polyurethanschaum-Herstellung verwendet werden kann, wird als Lösungsmittel für den TEDA-Quench bevorzugt ein Alkohol (z. B. Ethylenglykol, 1,4-Butandiol, Dipropylenglykol) eingesetzt. Die Farbzahl einer so erhaltenen 33 Gew.-%igen TEDA-Lösung in Dipropylenglykol beträgt kleiner 150 APHA, insbesondere kleiner 100 APHA.

Zur erfindungsgemäßen Herstellung von reinem (kristallinem) TEDA wird als Lösungsmittel für den TEDA-Quench bevorzugt ein aliphatischer Kohlenwasserstoff, insbesondere ein gesättigter aliphatischer Kohlenwasserstoff mit 5 bis 8 C-Atomen (wie z. B. Pentan, Hexan, Heptan) verwendet. Die Kristallisation des reinen TEDAs aus der erfindungsgemäß hergestellten TEDA-Lösung kann nach den dem Fachmann bekannten Verfahren erfolgen. Die durch eine nachfolgende mehrstufige, oder bevorzugt einstufige, Kristallisation erhaltenen TEDA-Kristalle sind hochrein (Reinheit von im allgemeinen mindestens 99,5 Gew.-%, insbesondere mindestens 99,9 Gew.-%) und die Farbzahl einer 33 Gew.-%igen Lösung in Dipropylenglykol beträgt kleiner 50, insbesondere kleiner 30, APHA.

Die Einleitung des dampfförmigen TEDAs in das flüssige Lösungsmittel erfolgt in einem Quenchapparat, z. B. bevorzugt in einem Fallfilmkondensator (Dünnschicht-, Rieselfilm- oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDAs von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Das Lösungsmittel für den TEDA-Quench kann im einmaligen Durchlauf oder als Kreislauflösung eingesetzt werden.
Die Menge des verwendeten Lösungsmittels im TEDA-Quench ist nicht erfindungswesentlich und wird nach Zweckmäßigkeitsgesichtspunkten ausgewählt. Im allgemeinen wird so verfahren, dass, je nach Art des Lösungsmittels, Lösungen mit einem TEDA-Gehalt von ca. 1 bis 50 Gew.-%, bevorzugt 20 bis 40 Gew.-%, erhalten werden.

Im allgemeinen wird die Temperatur im TEDA-Quench durch Temperierung des eingesetzten Lösungsmittels und/oder des Quenchapparates auf 20 bis 100 °C, bevorzugt 30 bis 60 °C, eingestellt.

Der Absolutdruck im TEDA-Quench beträgt im allgemeinen 0,5 bis 1,5 bar.

Durch die partielle Verdampfung des eingesetzten Lösungsmittels im TEDA-Quench infolge der Wärmezufuhr des dampfförmigen TEDAs ist der Gasraum im Quenchapparat mit Lösungsmitteldampf gesättigt. Dadurch wird die Desublimation des dampfförmigen TEDAs und die dadurch bedingten Verstopfungsprobleme durch Feststoffablagerung in den Austragsleitungen deutlich reduziert oder völlig verhindert.

Das im erfindungsgemäßen Verfahren eingesetzte und zu verdampfende TEDA kann nach den bekannten Verfahren, z. B. durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl) -piperazin, N-(2-Aminoethyl)-piperazin, N,N'-Bis(2-Aminoethyl)-piperazin, Morpholin oder Mischungen hiervon an einem Katalysator [z. B. Metallpyrophosphate, Metallphosphate (wie Erdalkalimonohydrogenphosphat), Zeolithe, Zirkoniumphosphate, Al₂O₃, SiO₂, phosphorhaltiges TiO₂ oder ZrO₂] bei erhöhter Temperatur (im allgemeinen 250 bis 450 °C), erhalten werden. Üblicherweise beträgt hierbei der Druck 0,1 bis 50, insbesondere 0,1 bis 5, bar. Optional kann die Umsetzung in Gegenwart eines inerten polaren aprotischen Lösungsmittels (z. B. N-Alkylpyrrolidon (wie N-Methylpyrrolidon), Dioxan, THF, Dialkylformamid (wie Dimethylformamid), Dialkylacetamid (wie Dimethylacetamid)) und eines inerten Trägergases (z. B. N₂ oder Ar) durchgeführt werden.

Derartige Verfahren sind z. B. beschrieben in DT-A-24 42 929, US-A-3,297,701, DE-A-36 34 258, DE-A-1 745 627, DE-A-37 18 395, EP-A-111 928, EP-A-382 055, EP-A-842 935, EP-A-842 936, EP-A-831 096, EP-A-952 152 und US-A-5,741,906.

Gemäß einer bevorzugten Ausführungsform lässt sich das erfindungsgemäße Verfahren wie folgt ausführen:

Eine Mischung enthaltend TEDA, die z. B. als Reaktionsaustrag in einem kontinuierlichen Verfahren durch Umsetzung von Ethylendiamin und Piperazin in einem Gasphasenreaktor bei 320 bis 420 °C und 0,5 bis 1,5 bar in Gegenwart eines Verdünnungsmittels (z. B. Wasser), eines Trägergases (z. B. N₂ oder Ar) und eines Zeolithkatalysators erhalten wurde (z. B. gemäß der älteren deutschen Patentanmeldung Nr. 10061863.4 vom 12.12.00), wird in eine Destillationsapparatur mit einer Destillationskolonne mit z. B. ca. 15 theoretischen Stufen geleitet. Hier werden Leichtsieder (wie z. B. Ammoniak, Ethylamin, Wasser) bei einer Kopftemperatur von 95 bis 120 °C und einem Druck von im allgemeinen 500 mbar bis 1,5 bar über Kopf abgetrennt. Der Sumpfablauf wird in eine weitere Destillationskolonne mit z. B. ca. 30 theoretischen Stufen gepumpt. Bei einer Kopftemperatur von 140 bis 160 °C und einem Druck von im allgemeinen 500 mbar bis 1,5 bar wird in dieser Kolonne Piperazin über Kopf abgetrennt und optional wieder zurück zum Synthesereaktor gefahren.

In dieser Kolonne erfolgt die erfindungsgemäße Zugabe eines flüssigen Lösungs- oder Verdünnungsmittels (z. B. NMP), in dessen Gegenwart das TEDA später verdampft wird (siehe unten). Vorteilhaft ist die Zugabe des Lösungs- oder Verdünnungsmittels in den Sumpf der Kolonne. Der Sumpfablauf enthaltend TEDA und das Lösungsoder Verdünnungsmittel wird in eine weitere Destillationskolonne mit z. B. ca. 25 theoretischen Stufen gepumpt. Bei einem Druck von im allgemeinen 500 mbar bis 1,5 bar wird in dieser Kolonne das in der vorherigen Kolonne zugesetzte Lösungs- oder Verdünnungsmittel aus dem Seitenabzug abgetrennt und optional wieder zurück zur vorausgegangenen Kolonne gefahren oder zusammen mit den Schwersiedern über den Sumpfablauf ausgeschleust. Am Kopf der Kolonne wird TEDA mit einer Reinheit von größer 95 Gew.-%, insbesondere größer 97 Gew.-%, über einen Teilkondensator dampfförmig abgezogen und in einem Fallfilmkondensator in einem Lösungsmittel (z. B. Pentan, Dipropylenglykol) bei einer Temperatur von im allgemeinen 30 bis 100 °C, bevorzugt 30 bis 60 °C, direkt schockartig abgekühlt und gleichzeitig gelöst (TEDA-Quench).

### Beispiele

### Beispiel 1 (Vergleichsbeispiel):

Die Versuche wurden in einem mit elektrischen Heizbändern beheizten 4 l (Katalysatorvolumen) Salzbadreaktor (Rohrbündel aus 7 Rohren, Innendurchmesser 21 mm, Länge 2 m) aus rostfreiem Stahl durchgeführt. Die Rohrleitungen für den Reaktorfeed, Reaktoraustrag und den Destillationsteil waren zum Teil als Doppelmantelrohre ausgeführt und ölbeheizt. Die Anlagenteile waren schutzbeheizt und wurden durch Einsatz verschiedener Heizkreisläufe an die jeweils erforderliche Temperatur individuell angepasst. Als Katalysator wurde ein Zeolith in Form von Strängen (Durchmesser ca. 2 mm, Länge ca. 30 mm) verwendet (Katalysatorschüttung).

Das Einsatzgut von 1300 g/h sowie 3 Nl/h (Nl = Normliter = auf Normalbedingungen umgerechnetes Volumen) Stickstoff wurden bei Normaldruck in den auf 350 °C beheizten Salzbadreaktor geleitet (Katalysatorbelastung: 1 kg Einsatzgut pro 1 Kat. (Schüttvolumen) und pro h)).

Das Einsatzgut hatte folgende Zusammensetzung (Angaben in Gew.-%):

| | |
|---|---|
| Ethylendiamin (EDA) | 30 % |
| Piperazin (PIP) | 20 % |
| Wasser | 50 % |

Das dampfförmige Reaktionsprodukt wurde in einem Quench mit Kreislaufflüssigkeit, die aus zuvor erhaltenem flüssigem Reaktionsprodukt bestand (siehe unten), bei 80 °C kondensiert (= Reaktionsautrags-Quench).

Die Analyse des Kondensats ergab folgende Zusammensetzung (Angaben in Gew.-%):

| | |
|---|---|
| Ammoniak | 3 % |
| Piperazin (PIP) | 17 % |
| Triethylendiamin (TEDA) | 23 % |
| Wasser | 54 % |
| Rest | Schwersieder und andere Nebenprodukte |

Die nicht kondensierten Anteile wurden nach einem Gas/Flüssig-Abscheider in die Destillationskolonne K 200 abgeleitet.

Ein Teil des flüssigen Reaktionsproduktes wurde gekühlt und als Flüssigkeitskreislauf (für den Reaktionsautrags-Quench) verwendet, ein anderer Teil wurde kontinuierlich mittels einer Pumpe in eine Destillationskolonne (K 200) gepumpt. Die Glaskolonne mit einem Durchmesser von 50 mm war mit 30 Glockenböden ausgerüstet. Das Rücklaufverhältnis betrug etwa 1 : 1.

Die Leichtsieder (Ammoniak, Ethylamin, Wasser) wurden bei Normaldruck und einer Kopftemperatur von 96 °C am Kopf der Kolonne flüssig abgezogen.

Die Analyse der Leichtsiederfraktion ergab folgende Zusammensetzung (Angaben in Gew.-%):

| | |
|---|---|
| Ammoniak | 13 % |
| Ethylamin | 2 % |
| Piperazin (PIP) | 2 % |
| Wasser | 83 % |

Der Sumpfablauf der Destillationskolonne wurde bei 155 °C kontinuierlich in eine nachfolgende Destillationskolonne K 300 gepumpt. Die Glaskolonne mit einem Durchmesser von 50 mm war mit 60 Glokkenböden ausgerüstet. Das Rücklaufverhältnis betrug etwa 10 : 1. Piperazin wurde bei Normaldruck und einer Kopftemperatur von 150 °C am Kopf der Kolonne flüssig abgezogen und zum Reaktor zurückgefahren.

Die Analyse der Destillatfraktion ergab folgende Zusammensetzung (Angaben in Gew.-%):

| | |
|---|---|
| Piperazin (PIP) | 93 % |
| Triethylendiamin (TEDA) | 6 % |
| Wasser | 1 % |

Der Sumpfablauf der Destillationskolonne wurde bei 184 °C kontinuierlich in eine nachfolgende Destillationskolonne K 400 gepumpt. Die Analyse des Sumpfablaufs ergab folgende Zusammensetzung (Angaben in Gew.-%):

| | |
|---|---|
| Piperazin (PIP) | 0,2 % |
| Triethylendiamin (TEDA) | 83 % |
| Rest | Schwersieder und andere Nebenprodukte |

Die Glaskolonne K 400 mit einem Durchmesser von 50 mm war mit 50 Glockenböden ausgerüstet. Das Rücklaufverhältnis betrug etwa 8 : 1. Die Schwersieder wurden kontinuierlich bei 230 °C über den Kolonnensumpf ausgeschleust, die Vorlauftemperatur des ölbeheizten Verdampfers betrug 260 °C.

Am Kopf der Kolonne wurde TEDA dampfförmig abgezogen und bei ca. 30 °C im flüssigen Lösungsmittel Pentan (Mischung aus 80 Gew.-% n-Pentan und 20 Gew.-% iso-Pentan) schockartig abgekühlt und gleichzeitig gelöst (= TEDA-Quench). Für den TEDA-Quench wurde ein Fallfilmkondensator (Rieselfilm- oder Fallstromkondensator) eingesetzt, bei dem dampfförmiges TEDA von oben eingeleitet wurde. Die Pentanzufuhr erfolgte tangential am Kopf des Fallfilmkondensators.

Die resultierende Lösung hatte folgende Zusammensetzung (Angaben in Gew.-%):

| | |
|---|---|
| Pentan | 91,8 % |
| Piperazin (PIP) | 0,2 % |
| Triethylendiamin (TEDA) | 8 % |

Nach der Abtrennung von Pentan durch Verdampfungskristallisation bei 25 °C unter Stickstoff wurde TEDA in einer Reinheit von mindestens 95 Gew.-% erhalten.

Das so gewonnene TEDA wies bezüglich seiner Farbe und seines Geruches ungenügende Eigenschaften auf und war deshalb nicht marktfähig.

Das erhaltene TEDA wies einen Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen auf.

Die erforderliche hohe Temperatur im Abtriebsteil der Destillationskolonne K 400 (Produkttemperatur bis zu 230 °C) führte zu erheblichen thermischen Belastungen des TEDAs und der Schwersieder und dadurch zur Bildung unerwünschter Zersetzungsprodukte.

Durch Bilanzierung der Zulauf- und Ablaufströme der Kolonne kann auf eine Piperazin-Quelle im Sumpf der K 400 geschlossen werden. Als PIP-Quelle im Sumpf der K 400 wird die Zersetzung von Hochsiedern (z. B.: Aminoethylpiperazin) angenommen.

### Beispiel 2 (erfindungsgemäß):

Durchführung des Versuchs wie in Beispiel 1 beschrieben, jedoch unter Zugabe des Lösungsmittels N-Methyl-2-pyrrolidon in die K 300.

In den Sumpf der Kolonne K 300 wurden 200 g/h N-Methyl-2-pyrrolidon eingeleitet.

Der Sumpfablauf der Destillationskolonne wurde bei 185 °C kontinuierlich in die nachfolgende Destillationskolonne K 400 gepumpt.

Die Analyse des Sumpfablaufs ergab folgende Zusammensetzung (Angaben in Gew.-%):

| | |
|---|---|
| Piperazin (PIP) | 0,03 % |
| Triethylendiamin (TEDA) | 53 % |
| N-Methyl-2-pyrrolidon | 43 % |
| Rest | Schwersieder und andere Nebenprodukte |

Die Glaskolonne K 400 mit einem Durchmesser von 50 mm war mit 50 Glockenböden ausgerüstet. Das Rücklaufverhältnis betrug etwa 8 : 1. Das Lösungsmittel N-Methyl-2-pyrrolidon und die Schwersieder wurden kontinuierlich bei 200 °C über den Kolonnensumpf ausgeschleust, die Vorlauftemperatur des ölbeheizten Verdampfers betrug 230 °C. Am Kopf der Kolonne wurde TEDA dampfförmig (gasförmig) abgezogen und bei ca. 30 °C im Lösungsmittel Pentan (Mischung aus 80 Gew.-% n-Pentan und 20 Gew.-% iso-Pentan) schockartig abgekühlt und gleichzeitig gelöst (= TEDA-Quench). Für den TEDA-Quench wurde ein Fallfilmkondensator (Rieselfilm- oder Fallstromkondensator) eingesetzt, bei dem dampfförmiges TEDA von oben eingeleitet wurde. Die Pentanzufuhr erfolgte tangential am Kopf des Fallfilmkondensators. Die resultierende Lösung hatte folgende Zusammensetzung (Angaben in Gew.-%):

| | |
|---|---|
| Pentan | 94,99 % |
| Piperazin (PIP) | 0,01 % |
| Triethylendiamin (TEDA) | 5 % |

Nach der Abtrennung von Pentan durch Verdampfungskristallisation bei 25 °C unter Stickstoff wurde TEDA in einer Reinheit von mindestens 99,5 Gew.-% erhalten.

Eine 33 Gew.-%ige Lösung des erhaltenen TEDAs in Dipropylenglykol (DPG) besaß eine APHA-Farbzahl von 26.

Das erhaltene TEDA wies keinen Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen auf.

### Beispiel 3 (erfindungsgemäß):

Bei Durchführung des Versuchs wie in Beispiel 2 beschrieben, jedoch unter Verwendung von Dipropylenglykol (DPG) anstelle von Pentan als Lösungsmittel für den TEDA-Quench und ohne anschließende Kristallisation des TEDAs aus dem Lösungsmittel wurde folgendes Ergebnis erhalten.

Zusammensetzung der TEDA/DPG-Lösung (Angaben in Gew.-%):

| | |
|---|---|
| Piperazin (PIP) | 0,05 % |
| Triethylendiamin (TEDA) | 33 % |
| Dipropylenglykol | 66,95 % |

Diese TEDA/DPG-Lösung besaß eine APHA-Farbzahl von 55 und kann direkt als Katalysator bei der Herstellung von Polyurethanen eingesetzt werden.

Die erhaltene TEDA/DPG-Lösung wies keinen Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen auf.

## Patentansprüche

1. Verfahren zur Herstellung einer Lösung von reinem Triethylendiamin (TEDA), **dadurch gekennzeichnet, dass** man TEDA aus einer Mischung enthaltend ein Lösungsmittel oder ein Verdünnungsmittel, wobei das Lösungs- oder Verdünnungsmittel bei Normaldruck einen Siedepunkt im Bereich von 175 bis 250 °C aufweist, verdampft und das dampfförmige TEDA in ein flüssiges Lösungsmittel einleitet.

2. Verfahren nach Anspruch 1, **dadurch gekennzeichnet, dass** man das dampfförmige TEDA am Kopf oder in einem Seitenabzug einer Destillations- oder Rektifikationskolonne erhält.

3. Verfahren zur Herstellung von reinem Triethylendiamin (TEDA), **dadurch gekennzeichnet, dass** man eine Lösung von reinem TEDA gemäß den Ansprüchen 1 oder 2 herstellt und anschließend das TEDA aus dieser Lösung auskristallisiert.

4. Verfahren nach einem der vorhergehenden Ansprüche, **dadurch gekennzeichnet, dass** das Lösungs- oder Verdünnungsmittel, das die Mischung enthält, aus der das TEDA verdampft wird, aus der Gruppe polare aprotische Lösungsmittel, cyclische oder acyclische Kohlenwasserstoffe, chlorierte aliphatische Kohlenwasserstoffe, aromatische Kohlenwasserstoffe, chlorierte aromatische Kohlenwasserstoffe, Alkohole, Amine, N-Alkylamide und deren Gemische, ausgewählt wird.

5. Verfahren nach Anspruch 4, **dadurch gekennzeichnet, dass** das polare aprotische Lösungsmittel aus der Gruppe der N-Alkyl-2-pyrrolidone, Ether, Ketone, Lactone, Sulfoxide, Carbonsäureester, Nitrile und Harnstoffe ausgewählt wird.

6. Verfahren nach einem der vorhergehenden Ansprüche, **dadurch gekennzeichnet, dass** das Lösungs- oder Verdünnungsmittel, das die Mischung enthält, aus der das TEDA verdampft wird, bei Normaldruck einen Siedepunkt im Bereich von 180 bis 230 °C aufweist.

7. Verfahren nach einem der vorhergehenden Ansprüche, **dadurch gekennzeichnet, dass** man zur Verdampfung des TEDAs einen Dünnschichtverdampfer oder Fallfilmverdampfer einsetzt.

8. Verfahren nach einem der vorhergehenden Ansprüche, **dadurch gekennzeichnet, dass** das flüssige Lösungsmittel in welches das dampfförmige TEDA eingeleitet wird, aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt wird.

9. Verfahren nach Anspruch 8, **dadurch gekennzeichnet, dass** man als flüssiges Lösungsmittel Pentan oder Dipropylenglykol einsetzt.

10. Verfahren nach einem der vorhergehenden Ansprüche, **dadurch gekennzeichnet, dass** das dampfförmige TEDA zur Einleitung in das flüssige Lösungsmittel eine Reinheit von größer 95 Gew.-% besitzt.

11. Verfahren nach einem der vorhergehenden Ansprüche, **dadurch gekennzeichnet, dass** das aus einer Mischung enthaltend ein Lösungs- oder Verdünnungsmittel zu verdampfende TEDA durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl)-piperazin, N-(2-Aminoethyl)-piperazin, N,N'-Bis(2-Aminoethyl)-piperazin, Morpholin oder Mischungen hiervon an einem Katalysator bei erhöhter Temperatur erhalten wurde.

12. Verfahren nach Anspruch 11, **dadurch gekennzeichnet, dass** es sich bei dem Katalysator um ein Metallphosphat oder einen Zeolith handelte.

13. Verfahren nach den Ansprüchen 11 oder 12, **dadurch gekennzeichnet, dass** man die Umsetzung bei Temperaturen von 250 bis 450 °C in der Gasphase durchführte.

## Claims

1. A process for the preparation of a solution of pure triethylenediamine (TEDA), which comprises evaporating TEDA from a mixture comprising a solvent or diluent, where the solvent or diluent has a boiling point at atmospheric pressure in the range from 175 to 250°C, and introducing the vapor-form TEDA into a liquid solvent.

2. A process as claimed in claim 1, wherein the vapor-form TEDA is obtained at the top or at a side take-off of a distillation or rectification column.

3. A process for the preparation of pure triethylenediamine (TEDA), which comprises preparing a solution of pure TEDA as claimed in claim 1 or 2 and subsequently crystallizing the TEDA out of this solution.

4. A process as claimed in one of the preceding claims, wherein the solvent or diluent present in the mixture from which the TEDA is evaporated is selected from the group consisting of polar aprotic solvents, cyclic or acyclic hydrocarbons, chlorinated aliphatic hydrocarbons, aromatic hydrocarbons, chlorinated aromatic hydrocarbons, alcohols, amines, N-alkylamides and mixtures thereof.

5. A process as claimed in claim 4, wherein the polar aprotic solvent is selected from the group consisting of N-alkyl-2-pyrrolidones, ethers, ketones, lactones, sulfoxides, carboxylic acid esters, nitriles and ureas.

6. A process as claimed in one of the preceding claims, wherein the solvent or diluent present in the mixture from which the TEDA is evaporated has a boiling point at atmospheric pressure in the range from 180 to 230°C.

7. A process as claimed in one of the preceding claims, wherein a thin-film evaporator or falling-film evaporator is employed for the evaporation of the TEDA.

8. A process as claimed in one of the preceding claims, wherein the liquid solvent into which the vapor-form TEDA is passed is selected from the group consisting of cyclic or acyclic hydrocarbons, chlorinated aliphatic hydrocarbons, aromatic hydrocarbons, alcohols, ketones, aliphatic carboxylic acid esters, aliphatic nitriles and ethers.

9. A process as claimed in claim 8, wherein the liquid solvent employed is pentane or dipropylene glycol.

10. A process as claimed in one of the preceding claims, wherein the vapor-form TEDA for passing into the liquid solvent has a purity of greater than 95% by weight.

11. A process as claimed in one of the preceding claims, wherein the TEDA to be evaporated from a mixture comprising a solvent or diluent has been obtained by reaction of monoethanolamine, diethanolamine, triethanolamine, ethylenediamine, diethylenetriamine, triethylenetetraamine, piperazine, N-(2-hydroxyethyl)piperazine, N,N'-bis(2-hydroxyethyl)piperazine, N-(2-aminoethyl)piperazine, N,N'-bis(2-aminoethyl)piperazine, morpholine or mixtures thereof on a catalyst at elevated temperature.

12. A process as claimed in claim 11, wherein the catalyst is a metal phosphate or a zeolite.

13. A process as claimed in claim 11 or 12, wherein the reaction is carried out in the gas phase at a temperature of from 250 to 450°C.

## Revendications

1. Procédé de préparation d'une solution de triéthylènediamine (TEDA) pure, **caractérisé en ce que** l'on évapore la TEDA à partir d'un mélange contenant un solvant ou un diluant, le solvant ou le diluant présentant un point de fusion sous la pression normale de 175°C à 250°C, et que l'on introduit la TEDA en phase vapeur dans un solvant liquide.

2. Procédé selon la revendication 1, **caractérisé en ce que** l'on obtient la TEDA en phase vapeur au niveau de la tête ou d'une évacuation latérale d'une colonne de distillation ou de rectification.

3. Procédé de préparation de triéthylènediamine (TEDA) pure, **caractérisé en que** l'on prépare une solution de TEDA pure selon les revendications 1 ou 2 et que l'on recristallise ensuite la TEDA dans cette solution.

4. Procédé selon l'une quelconque des revendications précédentes, **caractérisé en ce que** le solvant ou le diluant contenu dans le mélange à partir duquel on évapore la TEDA, est choisi dans le groupe formé par les solvants polaires aprotiques, les hydrocarbures cycliques ou acycliques, les hydrocarbures aliphatiques chlorés, les hydrocarbures aromatiques, les hydrocarbures aromatiques chlorés, les alcools, les amines, les N-alkylamides et leurs mélanges.

5. Procédé selon la revendication 4, **caractérisé en ce que** le solvant polaire aprotique est choisi dans le groupe formé par les N-alkyl-2-pyrrolidones, les éthers, les cétones, les lactones, les sulfoxydes, les esters d'acides carboxyliques, les nitriles et les urées.

6. Procédé selon l'une quelconque des revendications précédentes, **caractérisé en ce que** le solvant ou le diluant contenu dans le mélange à partir duquel on évapore la TEDA, présente un point de fusion sous la pression normale de 180°C à 230°C.

7. Procédé selon l'une quelconque des revendications précédentes, **caractérisé en ce que** l'on met en oeuvre pour l'évaporation de la TEDA un évaporateur à couche mince ou un évaporateur à couche descendante.

8. Procédé selon l'une quelconque des revendications précédentes, **caractérisé en ce que** le solvant liquide, dans lequel on introduit la TEDA en phase vapeur, est choisi dans le groupe formé par les hydrocarbures cycliques ou acycliques, les hydrocarbures aliphatiques chlorés, les hydrocarbures aromatiques, les alcools, les cétones, les esters d'acides carboxyliques aliphatiques, les nitriles et les éthers aliphatiques.

9. Procédé selon la revendication 8, **caractérisé en ce que** l'on met en oeuvre, en tant que solvant liquide, le pentane ou le dipropylèneglycol.

10. Procédé selon l'une quelconque des revendications précédentes, **caractérisé en ce que** la TEDA en phase vapeur présente, pour son introduction dans le solvant liquide, une pureté supérieure à 95% en poids.

11. Procédé selon l'une quelconque des revendications précédentes, **caractérisé en ce que** l'on obtient la TEDA à évaporer à partir d'un mélange contenant un solvant ou un diluant en faisant réagir de la monoéthanolamine, de la diéthanolamine, de la triéthanolamine, de l'éthylènediamine, de la diéthylènetriamine, de la triéthylènetétramine, de la pipérazine, de la N-(2-hydroxyéthyl)-pipérazine, de la N,N'-bis(2-hydroxyéthyl)-pipérazine, de la N-(2-aminoéthyl)-pipérazine, de la N,N'-bis(2-aminoéthyl)-pipérazine, de la morpholine ou des mélanges de celles-ci sur un catalyseur à haute température.

12. Procédé selon la revendication 11, **caractérisé en ce que** le catalyseur est un phosphate métallique ou une zéolithe.

13. Procédé selon les revendications 11 ou 12, **caractérisé en ce que** l'on réalise la réaction en phase gazeuse à des températures allant de 250°C à 450°C.
